# EUROPÄISCHE PATENTSCHRIFT

(11) **EP 2 799 017 B1**
(45) Veröffentlichungstag und Bekanntmachung des Hinweises auf die Patenterteilung: **02.11.2016**
(21) Anmeldenummer: 14001519.9
(22) Anmeldetag: 30.04.2014
(51) Int. Cl.: A61B 17/06

(54) **Abgabeverpackung für ein chirurgisches Nahtmaterial und Verfahren zum Verpacken desselben**
Dispensing package for a surgical suture material and method for packing the same
Emballage distributeur pour un matériel de suture chirurgical et son procédé d'emballage

(30) Priorität: 04.05.2013 DE 102013007721
(43) Veröffentlichungstag der Anmeldung: 05.11.2014
(73) Patentinhaber: Catgut GmbH, 08258 Markneukirchen (DE)
(72) Erfinder: Bernhardt, Gernot, 08239 Bergen (DE)
(74) Vertreter: Helge, Reiner

(56) Entgegenhaltungen:
- EP-A1- 1 354 796
- EP-A1- 1 498 076
- US-A- 5 284 240
- US-A- 6 047 815

## Beschreibung

Die Erfindung betrifft eine Abgabeverpackung für ein chirurgisches Nahtmaterial und ein Verfahren zum Verpacken desselben, wobei das chirurgische Nahtmaterial aus einem Nahtmaterialstück und einer an einem Ende oder zwei an seinen Enden befestigten Nadeln besteht, umfassend eine Umverpackung und einen Fadenträger.

Verpackungen für chirurgisches Nahtmaterial sind in vielfältigen Ausführungen bekannt. Es gibt zwei Arten von Verpackungen die herkömmlich für chirurgische Fäden und Nadeln verwendet werden. Zum einen sind mappenartige Papierverpackungen bekannt, bei denen ein Kartonmaterial medizinischer Qualität gefaltet und in eine Vielzahl von Karten geschnitten wird. Der Faden wird auf eine Karte gewickelt und die Verpackung in die gewünschte Konfiguration gefalten.
Zum anderen werden Fadenverpackungen aus Kunststoff verwendet. Diese Abgabeverpackungen weisen einen geschlossenen Wickelkanal auf und haben eine vorzugsweise ovale Form mit einer inneren und einer äußeren Wandung, die den ovalen Wickelkanal bilden. Die Verpackungen werden auf einem Wickelgestell angeordnet und die Fäden werden dann in den Wickelkanal gewickelt. Fadenverpackungen weisen Nadelablageelemente zum Halten und Fixieren der atraumatischen Nadeln auf, sofern eine Nadel an den Fäden angeordnet ist.
Derartige Halterungen können aus Schaumstoff oder anderen Rückhaltestrukturen bestehen.

Die Fäden werden je nach Länge in mehreren Wickellagen, gegebenenfalls mit unterschiedlichen Wickelradien in den Wickelkanal eingebracht. Der Wickelkanal umschließt einen Aufnahmeraum, in dem die Nadeln fixiert sind Die Erfindung wird im Anspruch 1 definiert und weitere Ausführungsformen finden sich in den abhängigen Ansprüchen.

Aus der DE 600 32 702T2 ist eine Verpackung für chirurgische Fäden mit einem Basiselement, mit einer Außenwand, die sich nach oben vom Umfangsbereich des Basiselements erstreckt und mit einer Vielzahl von Abstandselementen, die sich radial nach innen erstrecken und mit einer Vielzahl einseitig angehängter Abdecktürelemente, die sich radial nach außen von der inneren Spurwand erstrecken, bekannt. Die Abdecktürelemente sind über Filmscharniere an der inneren Spurwand mit einem geringeren Winkel als 90 Grad befestigt. Die Abdecktürelemente bilden zusammen mit dem Basiselement einen Fadenkanal, in dem der Faden lose gehalten wird.

Aus der DE 690 23 852T2 ist eine Nahtmaterialverpackung bekannt, die die Hauptoberflächen einer Rinne für die Wicklung des Nahtmaterials festlegt, mit einer Anzahl von Klappen, die schwenkbar an der Rinne für die Nahtmaterialwicklung angebracht sind, um das Nahtmaterial in der Rinne zu umschließen und mit einer Einrichtung zum Festhalten in der geschlossenen Stellung. Nach dem Einwickeln des Nahtmaterials in die offene Rinne werden die Klappen über die offene Rinne geschwenkt, wobei sie einrasten und die Rinne verschließen.

Die DE 689 21 523T1 beschreibt eine ovale Verpackung für Nahtmaterial. Die geformte zweiteilige Nahtmaterialverpackung bildet einen ovalen Kanal, in dem das Nahtmaterial aufgewickelt ist, wobei der Boden des Kanals und die Innenwand des Kanals mindestens teilweise durch alternierende Wandteile gebildet werden, welche in den ersten und zweiten Packungsteilen geformt sind und die im ersten Teil gebildeten Wandteile gegenüber den angehobenen Bodenbereichen ausgerichtet sind und die im zweiten Teil geformten Wandabschnitte gegenüber dem abgesenkten Bodenbereich ausgerichtet sind.

Die DE 103 20 463A1 beschreibt einen Nahtmaterialdispenser, der einen Wickelkörper aufweist, der einen umlaufenden Wickelkanal zur Aufnahme des Nahtmaterials enthält. Der Wickelkörper ist ein einstückiges Kunststoffteil. Eine der Seitenwände des Wickelkanals ist schwenkbar, so dass sie geschwenkt werden kann, um den Wickelkanal zu schließen.

Die DE 197 44 5431 beschreibt einen Materialspender, der zwei Platten aus einer Hartfolie enthält und die mit Ausformungen ineinandergreifen und übereinanderliegend befestigt sind. Die Platten haben nach außen divergierende Randstreifen, die eine keilförmige Rinne bilden. Durch Drehen der Platten um die Mittelachse wird ein Nahtmaterialfaden in die Rinne gewickelt. Dieser Faden gelangt durch den Spalt am Boden der Rinne in einen Aufnahmeraum. Durch Ziehen an der Nadel, die an einem Ende des Fadens vorgesehen ist, wird der Wickel aus dem Aufnahmeraum herausgezogen.

Die DE 693 10 983T2 beschreibt eine ovale Verpackung für Nahtmaterial mit einem rotierenden Rad. Die Verpackung mit einem ovalen Nahtmaterial-Wickelkanal und mit zwei gegenüberliegenden Längsabschnitten, die mit einem ersten und einem zweiten halbkreisförmigen Endabschnitt verbunden sind, weist zur Unterstützung der Abgabe des Nahtmaterials eine Radvorrichtung auf, wobei die Radvorrichtung an der Nahtmaterialverpackung gegenüber dem ersten halbrunden Endabschnitts drehbar befestigt ist.

Weiterhin bekannt ist aus der DE 699 31 879T2 eine Verpackung zur mehrsträngigen Aufbewahrung von chirurgischen Nahtmaterial, welches in Kombination einen gefalteten Behälter und mindestens ein nicht eingefädeltes Nahtmaterial umfaßt, wobei das Nahtmaterial ein Griffelement aufweist und der Behälter vier benachbarte Elemente umfaßt, die jeweils schwenkbar mit einem benachbarten Element verbunden sind, wobei zwei der Elemente ein Deckelement umfassen, wobei das Nahtmaterial faltbar in Kontakt mit den Stützelementen angeordnet ist. Die Deckelemente sind jeweils über einen Abschnitt des Nahtmaterials und eines der Stützelemente gefaltet, um ein Auseinanderwickeln des Nahtmaterials zu verhindern. Der Nahtmaterialbehälter kann aber auch mit eingefädeltem Nahtmaterial verwendet werden.

Die EP 1498076 A1 beschreibt eine Nähfadenpackung umfassend eine Nähfadenaufnahmefläche mit einer Mehrzahl von Sätzen von Nähfadenaufnahmeflächen-Vorsprüngen, die einem umlaufenden ringartigen Kanal bilden und in den der Nähfaden spiralförmig eingelegt ist, und einer Abdeckung, die die Nähfadenaufnahmefläche topfartig umschließt. Die Nähfadenaufnahmefläche weist einen zentralen Durchbruch zur Durchführung des Nähfadens mit der Nadel nach außen auf, wobei die Nadel außerhalb der Nähfadenpackung auf der Außenseite der Nähfadenaufnahmefläche fixiert wird. Nachteilig ist hier die aufwendige Ausbildung der Nähfadenpackung, die aus unterschiedlich ausgebildeten Einzelteilen besteht, wodurch bei der Herstellung derselben unterschiedliche Formen benötigt werden. Ein weiteres Problem liegt bei der Bestückung der Nähfadenpackung darin, dass der Nähfaden spiralartig in den ringartigen Kanal eingelegt werden muss, ohne dass es zu einem Herausrutschen des Fadens aus der kanalartigen Führung kommt.

Die US 6 047 815 A beschreibt eine Verpackung für ein Nahtmaterial mit einem gewundenen Kanal umfassend ein Basiselement und ein Abdeckelement, dessen umlaufender Außenbereich aus einzelnen mittels Filmscharnieren am Innenbereich des Abdeckelements befestigten Flächenelementen besteht. Diese decken den den Nähfaden aufnehmenden Kanal der Verpackung ab.

Die US 5 284 240 A beschreibt eine Nahtmaterialpackung zur Aufnahme von sterilen Nahtmaterial mit einer daran befestigten Nadel. Diese umfasst einen Nahtmaterialhalter, der ein flaches Unterteil und Mittel aufweisen, die auf einer Seite zur Aufnahme des Nahtmaterials und zur Fixierung der Nadel dienen. Das Nahtmaterial wird um die Peripherie des ovalen Halters herum gehalten. Nach dem Einlegen des Nähfadens und der Fixierung der Nadel werden die abschnittsweise scharnierartig bewegbaren Klappelemente geschlossen. Anschließend wird der Nahtmaterialhalter in eine Schutzhülle eingesteckt und diese zusammen mit dem Nahtmaterialhalter in einer sterilen Packung verpackt. Die äußere Packung umfasst ein Bodenpapier und eine Oberschicht, die zusammen im Kantenbereich heißversiegelt ist.

Die EP 1354796 A1 beschreibt ein Verfahren und eine Vorrichtung zur Beschickung von chirurgischen Nadeln in chirurgische Nahtpackungen. Das Verfahren und die Vorrichtung dient nicht zum Bestücken des Fadenträgers sondern nur der Fixierung der Nadeln in einem Nadelblockelement.

Die Nahtmaterialverpackungen gemäß des Standes der Technik sind zwar effektiv und zweckmäßig, doch es gibt Nachteile, die mit diesen Verpackungen verbunden sind. Zum einen sind diese Verpackungen konstruktiv aufwendig ausgebildet und damit nicht kostengünstig herstellbar. Zum anderen sind die mit dem Nahtmaterial verbundenen Nadeln innerhalb des Nahtmaterialträgers, d. h., innerhalb des Wickelkanals, angeordnet, wodurch die Nadeln nicht problemlos entnommen werden können.

Die Kunststoffträger nach dem Stand der Technik erfordern Präzisionsformteile und oft auch zeitaufwendige Verfahren, um ein Bündel Nahtmaterialien in den äußeren Kanälen anzulegen. Auch kann das Verschließen der Verpackung schwierig sein, da das Nahtmaterial oft derart elastisch ist, dass es aus seiner Wickelanordnung herausspringt, wenn es nicht gehalten wird.

Ein weiteres Problem bei faltbaren Papierverpackungen besteht darin, dass das Nahtmaterial oftmals in Form einer Acht eingelegt ist, wodurch es sich bei der Entnahme leicht verknoten kann.

Aufgabe der Erfindung ist es daher, eine Abgabeverpackung anzugeben, die die Mängel der Papierverpackung als auch der Kunststoffträger beseitigt, wobei diese in einfacher Art und Weise mit dem Nahtmaterial bewickelt und die Nadeln außerhalb des Wickelkanals zur einfachen und schnellen Entnahme fixiert werden können. Demgemäß soll die Abgabeverpackung einfach und kostengünstig herstellbar sein. Weiterhin ist es die Aufgabe der Erfindung, ein Verfahren anzugeben, mit dem das Nahtmaterial einschließlich der Nadeln effektiv in die Abgabeverpackung eingebracht werden kann.

Erfindungsgemäß wird die Aufgabe durch eine Abgabeverpackung für ein chirurgisches Nahtmaterial mit den Merkmalen des Anspruches 1 und ein Verfahren mit dem Merkmalen des Anspruches 5 gelöst. Bevorzugte Ausführungsformen der Abgabeverpackung sind in den Unteransprüchen sowie im nachfolgenden Ausführungsbeispiel beschrieben.

Die erfindungsgemäße Abgabeverpackung für ein chirurgisches Nahtmaterial, das aus einem Nahtmaterialstück und einer an einem Ende oder an zwei Enden befestigte Nadeln besteht, umfaßt eine Umverpackung und einen Fadenträger, wobei der Fadenträger aus einer unteren und einer oberen Platte mit je einem umlaufenden ringartigen oder ovalen Innenbereich besteht und die zum Zusammenbau ineinander steckbar sind. Auf der der Fadenlage zugewandten Seite des umlaufenden ringartigen oder ovalen Innenbereichs sind mindestens zwei sich gegenüberliegende Erhebungen angeordnet.

Der ringartige oder ovale Innenbereich der unteren Platte ist größer als der umlaufende ringartige oder ovale Innenbereich der oberen Platte ausgebildet, wodurch die beiden Platten zusammensteckbar sind und den Fadenträger bilden.

Die am Nahtmaterialstück befestigte Nadel bzw. Nadeln sind außerhalb des Fadenträgers an einer Lasche der Umverpackung fixierbar.

Die Umverpackung und der Fadenträger können aus unterschiedlichen Materialien bestehen, vorzugsweise besteht die Umverpackung aus Papier oder Pappe und der Fadenträger aus einem Kunststoff.

Die untere und die obere Platte des Fadenträgers sowie die Umverpackung weisen mindestens einen Durchbruch auf, wobei die jeweiligen Durchbrüche miteinander korrespondieren und während des Wickelvorganges und des Faltvorganges der Umverpackung diese auf der Wickelvorrichtung fixieren.

Anhand eines Ausführungsbeispiels soll die Erfindung näher beschrieben werden. Es zeigen
Figur 1 - Fadenträger
Figur 2 - Einzelheit zum Fadenträger
Figur 3 - Umverpackung.

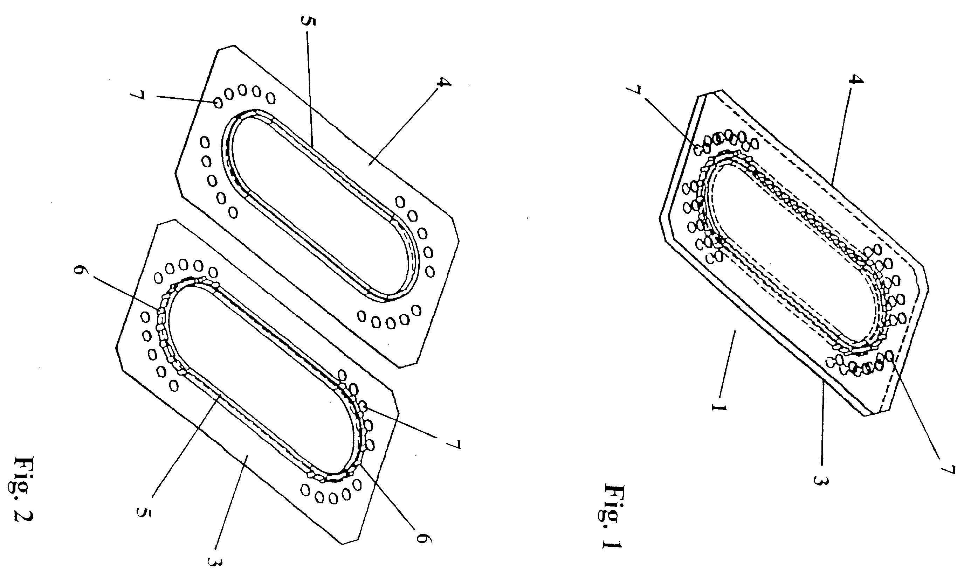

Die Figur 1 zeigt eine Explosivdarstellung des Fadenträgers **1.** Die Figur 2 zeigt als Einzelheit die untere **3** und die obere Platte **4** des Fadenträgers **1.** Sowohl die untere Platte **3** als auch die obere Platte **4** weisen einen ovalen Ringbereich auf, die zum Zusammenbau ineinander steckbar sind.

Die untere Platte **3** weist auf der Außenseite ihres ovalen Ringbereichs **5** sich gegenüberliegende Erhebungen **6** auf. Die Erhebungen **6** dienen zur Verringerung der Reibung des chirurgischen Nahtmaterials an der Außenseite des Ringbereiches **5** der unteren Platte **3** beim Herausziehen des Nahtmaterials.

Die Ringbereich **5** der oberen Platte **4** ist in seinen Abmessungen kleiner ausgebildet als der Ringbereich **5** der unteren Platte **3.** Dadurch ist es möglich, die obere Platte **3** nach dem Bewickeln der unteren Platte **3** mit dem Nahtmaterial aufzustecken und beide Platten **3** und **4** zueinander zu fixieren.

Sowohl die untere Platte **3** als auch die obere Platte **4** weisen im Bereich der gegenüberliegenden Rundungen des ovalen Ringbereiches **5** Durchbrüche **7,** die zum Ringbereich **5** beabstandet sind und zur Aufnahme des Fadenträgers bzw. der unteren **3** und oberen Platte **4** auf die Wickelvorrichtung dienen, auf.

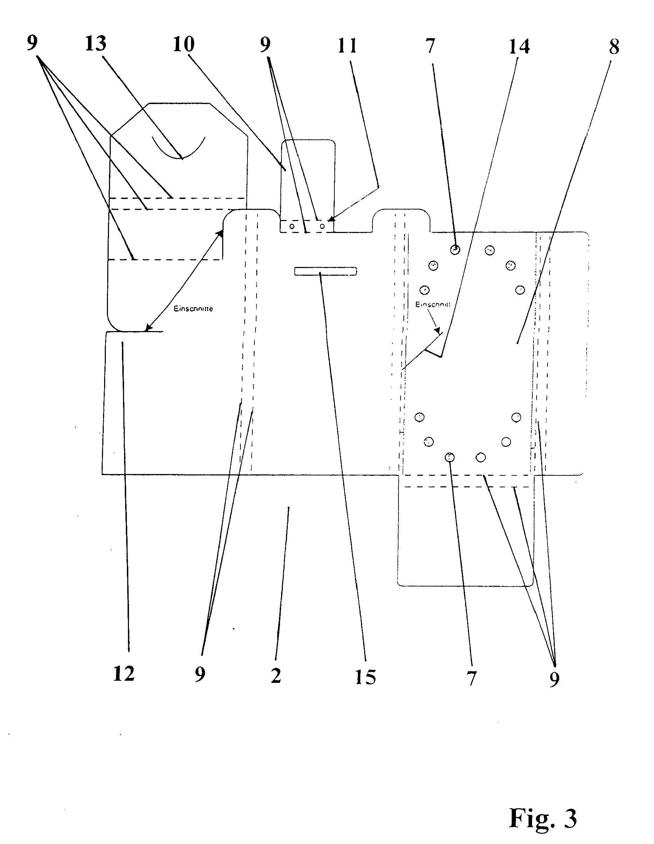

Die Figur **3** zeigt die Umverpackung **2** in einer ungefalteten Ansicht. Diese besteht aus einem Basisabschnitt **8,** welcher ebenfalls mit sich gegenüberliegenden Durchbrüchen **7** versehen ist. Die Umverpackung besteht aus weiteren um den Basisabschnitt faltbaren Abschnitten und der Lasche **10.** Die gestrichelt ausgeführten Linien **9** sind die Knicklinien, entlang derer die Umverpackung **2** gefaltet werden kann. Die im Basisabschnitt **8** angeordneten Durchbrüche **7** korrespondieren mit den Durchbrüchen **7** der unteren **3** und der oberen Platte **4** des Fadenträgers **1.**

Zum Verpacken eines mit zwei Nadeln versehenen chirurgischen Nahtmaterials wird in einem ersten Schritt die Umverpackung **2** im ungefalteten Zustand mit ihrem Basisabschnitt **8** auf die Dorne der Wickelvorrichtung aufgesetzt. Anschließend wird die untere Platte **3** auf den Basisabschnitt **8** und die Dorne der Wickeleinrichtung aufgesetzt und der Wickelvorgang gestartet.

Nach dem Wickeln des Nahtmaterials um den Ringbereich **5** der unteren Platte **3** wird die Nadel bzw. werden die Nadeln auf der Wickelvorrichtung mechanisch fixiert und die obere Platte **4** aufgesetzt, wobei diese mit ihrem Ringbereich **5** vom Ringbereich **5** der unteren Platte **3** umfasst wird.

Anschließend werden die beweglichen Dorne der Wickelvorrichtung soweit zurückgefahren, bis die Oberkanten der Dorne plan mit der oberen Platte **4** sind. Danach wird die Umverpackung **2** entlang der Linien **9** um den auf dem Basisabschnitt **8** angeordneten Fadenträger **1** gefaltet. Zum Entnehmen der Umverpackung **2** werden Dorne gänzlich gesenkt und die fixierten Nadeln mechanisch frei gegeben. Danach kann die Umverpackung der Wickelvorrichtung entnommen werden.
Zur Fixierung der Nadeln in der Umverpackung werden diese in den Lochungen **11** der Lasche **10** fixiert.
Zum Verschließen der Abgabeverpackung wird der Verschlußabschnitt **12** in den Einschnitt **14** und der Verschlußabschnitt **13** in die Öffnung **15** gesteckt.

### Bezugszeichenaufstellung

- 1 -: Fadenträger
- 2 -: Umverpackung
- 3 -: untere Platte
- 4 -: obere Platte
- 5 -: Ringbereich
- 6 -: Erhebungen
- 7 -: Durchbrüche
- 8 -: Basisabschnitt
- 9 -: Linien
- 10 -: Lasche
- 11 -: Lochung
- 12 -: Verschlußabschnitt
- 13 -: Verschlußabschnitt
- 14 -: Einschnitt
- 15 -: Öffnung

## Patentansprüche

1. Abgabeverpackung für ein chirurgisches Nahtmaterial, das aus einem Nahtmaterialstück und einer an einem Ende oder zwei an beiden Enden befestigten Nadeln besteht, mit einem Fadenträger, wobei der Fadenträger durch Zusammenstecken einer unteren und einer oberen Platte gebildet wird, wobei diese aus einer faltbaren Umverpackung (2) und einem aus einer unteren (3) und einer oberen Platte (4) zusammensteckbaren Fadenträger (1) besteht, wobei die untere (3), die obere Platte (4), die mit je einem umlaufenden ringartigen oder ovalen Ringbereich (5) versehen sind und mindestens ein Abschnitt der Umverpackung (2) mit mindestens einem Durchbruch (7), der zum Ringbereich nach außen hin beabstandet ist, zur Aufnahme auf die beweglichen Dorne einer Wickelvorrichtung versehen sind.

2. Abgabeverpackung nach Anspruch 1,
**dadurch gekennzeichnet, dass**
die Durchbrüche (7) der unteren (3) und der oberen Platte (4) des Fadenträgers (1) und der Umverpackung (2) miteinander korrespondierend ausgeführt sind.

3. Abgabeverpackung nach Anspruch 1 und 2,
**dadurch gekennzeichnet, dass**
nach dem Zusammenstecken der unteren Platte (3) und der oberen Platte (4) zum Fadenträger (1) die seitlichen Randbereiche offen ausgeführt sind.

4. Abgabeverpackung nach Anspruch 1 bis 3,
**dadurch gekennzeichnet, dass**
die am Nahtmaterialstück befestigte Nadel bzw. Nadeln außerhalb des Fadenträgers (1) an einer Lasche (10) der Umverpackung (2) fixierbar sind.

5. Verfahren zum Verpacken eines mit einer oder zwei Nadeln versehenen chirurgischen Nahtmaterials in einer Abgabeverpackung gemäß Anspruch 1 und mindestens einen der Ansprüche 2 bis 4, umfassend die Schritte:
- Platzieren der Umverpackung (2) auf einer Wickelvorrichtung durch Fixieren, des mit den Durchbrüchen (7) versehenen Abschnitts (8), auf vertikal beweglichen Dornen der Wickelvorrichtung,
- Aufsetzen der unteren Platte (3) auf die beweglichen Dorne der Wickelvorrichtung,
- Wickeln der mit Nadeln versehenen chirurgischen Nahtmaterialstücke, wobei diese außerhalb der beweglichen Dorne der Wickelvorrichtung zum Liegen kommen,
- Mechanisches Fixieren der Nadeln auf der Wickelvorrichtung,
- Aufsetzen der oberen Platte (4) auf die untere Platte (3), wobei der ringförmige oder ovale Innenbereich (5) der unteren Platte (3) den ringförmigen oder ovalen Bereich (5) der oberen Platte (4) umfaßt,
- Absenken der beweglichen Dorne, bis die Oberkanten der Dorne plan mit der oberen Platte (4) sind,
- Schließen der Umverpackung (2) durch Falten der einzelnen Bereiche der Umverpackung (2) und gänzliches Absenken der Dorne der Wickelvorrichtung, Lösen der Nadelfixierung auf der Wickelvorrichtung,
- Entnahme der Abgabeverpackung von der Wickelvorrichtung,
- Fixieren der Nadeln in der Lasche (10) der Umverpackung (2),
- Verschließen der Abgabeverpackung durch Einstecken des Verschlußabschnittes (12) in den Einschnitt (14) und des Verschlußabschnittes (13) in die Öffnung (15).

## Claims

1. The dispensing package for surgical suture material that consists of a piece of suture material and one needle attached to one end or two needles attached to both ends, with a thread carrier, wherein the thread carrier is formed by fitting together a lower and an upper 'plate, consisting of a foldable outer packaging (2) and a thread carrier (1) which can be assembled by a lower (3) and an upper plate (4), wherein the lower plate (3), the upper plate (4), each provided with a surrounding ring-shaped or oval ring area (5), are intended for uptake of the movable spikes of an angular device with one section of the outer packaging (2) minimum with a minimum of one opening (7) that is located in a distance to the outer ring area (5).

2. The dispensing package according to claim 1
**characterised in, that**
the openings (7) of the lower (3) and the upper plate (4) of the thread carrier (1) and the outer packaging (2) are executed correspondingly with each other.

3. The dispensing package according to claims 1 and 2,
**characterised in, that**
the lateral marginal areas are executed in an open manner after fitting together the lower plate (3) and the upper plate (4) with the thread carrier (1).

4. The dispensing package according to claims 1 to 3,
**characterised in, that**
the needle and/or needles attached to the piece of the suture material are fixable at the outer area of the thread carrier (1) at a strap (10) of the outer packaging (2).

5. The procedures for packaging of surgical suture material located in a dispensing package and provided with one or two needles according to claim 1 and at least one of claims 2 to 4, comprise the following steps:
- Placement of the outer packaging (2) onto an angular device by fixation of the section (8) provided with openings (7) on vertically movable spikes of the angular device;
- Positioning of the lower plate (3) onto the movable spikes of the angular device;
- Wrapping of the pieces of surgical suture material provided with needles, wherein those will come to rest in the area away from the movable spikes of the angular device;
- Mechanical fixation of the needles onto the angular device;
- Placement of the upper plate (5) onto the lower plate (3), wherein the ring-shaped or oval internal area (5) of the lower plate (3) comprises the ring-shaped or oval area (5) of the upper plate (4);
- Lowering of the movable spikes until the upper edges of the spikes are on an even level with the upper plate (4);
- Closure of the outer packaging (2) by folding of the individual areas of the outer packaging (2) and full lowering of the spikes of the angular device, loosening of needle fixation to angular device,
- Removal of dispensing package from the angular device;
- Fixation of needles to the strap (10) of the outer packaging (2),
- Closure of the dispensing packaging by insertion of the closing section (12) into the notch (14) and the closure section (13) into the opening (15).

## Revendications

1. Emballage distributeur destiné à du matériel de suture chirurgical comprenant un morceau de matériel de suture et d'une aiguille fixée à une extrémité ou de deux aiguilles fixées aux deux extrémités, avec un porte-fil, sachant que le porte-fil est formé d'un assemblage d'une plaque inférieure et supérieure, sachant qu'il est composé d'un suremballage (2) pliable et d'un porte-fil (1) pouvant être assemblé d'une plaque inférieure (3) et supérieure (4), sachant que la plaque inférieure (3), la plaque supérieure (4) qui présentent chacune une zone annulaire (5) périphérique ovale ou ronde et qui présentent au moins une section du suremballage (2) avec au moins une ouverture (7), qui est espacée vers l'extérieur de la zone annulaire (5), pour la réception sur les mandrins mobiles d'un dispositif d'enroulement.

2. Emballage distributeur selon la revendication 1,
**caractérisé en ce que**
les ouvertures (7) de la plaque inférieure (3) et supérieure (4) du porte-fil (1) et du suremballage (2) sont exécutées de manière à ce qu'elles correspondent.

3. Emballage distributeur selon les revendications 1 et 2,
**caractérisé en ce que**
après l'assemblage de la plaque inférieure (3) et de la plaque supérieure (4), les zones périphériques latérales sont ouvertes vers le porte-fil (1).

4. Emballage distributeur selon les revendications 1 à 3,
**caractérisé en ce que**
l'aiguille fixée au morceau de matériel de suture ou les aiguilles en dehors du porte-fil (1) peuvent être fixées à la languette (10) du suremballage (2).

5. Procédé pour l'emballage d'un matériel de suture chirurgien présentant une ou deux aiguilles dans un emballage distributeur selon la revendication 1 et au moins une des revendications 2 à 5, comprenant les étapes suivantes :
- Placement du suremballage (2) sur un dispositif d'enroulement par fixation, de la section (8) prévue avec les ouvertures (7) sur des mandrins mobiles verticalement du dispositif d'enroulement,
- Mise en place de la plaque inférieure (3) sur les mandrins mobiles du dispositif d'enroulement,
- Enroulement des morceaux de matériel de suture présentant des aiguilles, sachant qu'ils doivent être posés à l'extérieur des mandrins mobiles du dispositif d'enroulement,
- Fixation mécanique des aiguilles sur le dispositif d'enroulement,
- Mise en place de la plaque supérieure (4) sur la plaque inférieure (3), sachant que la zone intérieure annulaire ou ovale (5) comprend la plaque supérieure (4),
- Abaissement des mandrins mobiles jusqu'à ce que les bords supérieurs des mandrins soient plans avec la plaque supérieure (4),
- Fermeture du suremballage (2) en pliant les différentes zones du suremballage (2) et abaissement complet des mandrins du dispositif d'enroulement, desserrage de la fixation des aiguilles sur le dispositif d'enroulement,
- Retrait de l'emballage distributeur du dispositif d'enroulement,
- Fixation des aiguilles sur la languette (10) du suremballage (2),
- Fermeture de l'emballage distributeur en insérant la partie de fermeture (12) dans l'incision (14) et la partie de fermeture (13) dans l'ouverture (15).
